# EUROPÄISCHE PATENTSCHRIFT

(11) **EP 1 123 342 B1**
(45) Veröffentlichungstag und Bekanntmachung des Hinweises auf die Patenterteilung: **05.11.2003**
(21) Anmeldenummer: 99945981.1
(22) Anmeldetag: 14.08.1999
(51) Int. Cl.: C08J 3/14, C08L 3/12

(54) **VERFAHREN ZUR HERSTELLUNG VON SPHÄRISCHEN MIKROPARTIKELN, DIE GANZ ODER TEILWEISE AUS MINDESTENS EINEM WASSERUNLÖSLICHEN VERZWEIGUNGEN ENTHALTENDEN POLYGLUCAN BESTEHEN, SOWIE MIT DIESEM VERFAHREN ERHÄLTLICHE MIKROPARTIKEL**
METHOD FOR THE PRODUCTION OF SPHERICAL MICROPARTICLES CONSISTING TOTALLY OR PARTLY OF AT LEAST ONE WATER-INSOLUBLE POLYGLUCAN CONTAINING BRANCHES AND MICROPARTICLES PRODUCED ACCORDING TO SAID METHOD
PROCEDE DE PREPARATION DE MICROPARTICULES SPHERIQUES, CONSISTANT EN PARTIE OU EN TOTALITE EN AU MOINS UN POLYGLUCANE CONTENANT DES RAMIFICATIONS NON SOLUBLE DANS L'EAU, ET MICROPARTICULES OBTENUES A L'AIDE DUDIT PROCEDE

(30) Priorität: 28.08.1998 DE 19839216
(43) Veröffentlichungstag der Anmeldung: 16.08.2001
(73) Patentinhaber: Celanese Ventures GmbH, 65926 Frankfurt (DE)
(72) Erfinder: BENGS, Holger, D-60598 Frankfurt am Main (DE); GRANDE, Jürgen, D-65812 Bad Soden (DE)
(74) Vertreter: Luderschmidt, Schüler & Partner
(86) Internationale Anmeldenummer: EP9905976
(87) Internationale Veröffentlichungsnummer: WO00012590

(56) Entgegenhaltungen:
- EP-A- 0 327 490
- EP-A- 0 528 893
- WO-A-85/02772
- GB-A- 2 247 242

## Beschreibung

Die vorliegende Erfindung betrifft ein Verfahren zur Herstellung von sphärischen Mikropartikeln, die ganz oder teilweise aus mindestens einem wesserunlöslichen Verzweigungen enthaltenden Polyglucan bestehen, sowie mit diesem Verfahren erhältliche Mikropartikel.

Ein Verfahren zur Herstellung von sphärischen Mikropartikeln, die wasserlösliche lineare Polysaccharide (Amylose) enthalten, sind in der GB-A-2 247 242 offenbart.

Die EP-A-0 529 893 offenbart tehenfalls Mikropartikel aus wasserlöslichen Polysacchariden (Amylopektin) zum Gegenstand.

Verfahren zur Herstellung von sphärischen Mikropartikeln, die wasserunlösliche lineare Polysaccharide enthalten, sind in der prioritätsälteren nichtvorveröffentlichten deutschen Patentanmeldung Nr. 19737481.6 der Anmelderin beschrieben. Mit diesem Verfahran können sphärische Mikropartikel erhalten werden, die sich insbesondere durch eine hohe Einheitlichkeit in bezug auf ihre Gestalt und Vertellung des Durchmessers sowie gute mechanische Eigenschaften auszeichnen.
Aufgrund ihrer vergleichsweise regelmäßigen Beschaffenheit bei gleichzeitig guten mechanischen Eigenschaften können diese Mikropartikel für eine Vielzahl von Anwendungen eingesetzt werden.

Als Ausgangsmaterial werden für das dort beschriebene Verfahren lineare wasserunlösliche Polysaccharide wie z.B. Polyglucane eingesetzt, die keine Verzweigungen aufweisen bzw. für die mit herkömmlichen Meßmethoden keine Verzweigungen nachgewiesen werden können. Lineare Polysaccharide bzw. Polyglucane kommen jedoch in der Natur nicht in reiner Form vor. Sie müsser daher entweder aus nativen Quellen wie z.B. Stärke mittels aufwendiger Verfahren isoliert oder hergestellt werden oder über biotechnische Verfahren gewonnen werden.

Insbesondere die Aufreinigung von Polysacchariden natürlichen z.B. pflanzlichen Ursprungs zu Verbindungen mit der erforderlichen hohen Linearität ist vergleichsweise zeit- und kostenintensiv, so daß natürliche Quellen bisher nur bedingt einsetzbar sind. In Hinblick auf ihre allgemeine Verfügbarkeit und Kostengünstigkeit ist jedoch eine weite Nutzung der natürlichen Quellen wünschenswert.

Überraschenderweise wurde nun gefunden, daß auch bei Einsatz von Polyglucanen, die Verzweigungen enthalten, qualitativ zufriedenstellende sphärische Mikropartikel erhalten werden können, die für eine Vielzahl von Anwendungen erfolgreich eingesatzt werden können.

Somit betrifft die vorliegende Erfindung ein Verfahren zur Herstellung von sphärischen Mikropartikeln, die ganz oder teilweise aus mindestens einem wasserunlöslichen Polysaccharid bestehen, wobei das mindestens eine wasserunlösliche Polysaccharid in einem Lösungsmittel oder Lösungsmittelgemisch gelöst wird, die gebildete Lösung in ein Fällmittel oder Fällmittelgemisch eingebracht wird, das dabei entstehende Gemisch gegebenenfalls gekühlt wird und die gebildeten Mikropartikel abgetrennt werden, dadurch gekennzeichnet, daß das wasserunlösliche Polysaccharid ausgewählt ist unter einem Verzweigungen enthaltenden Polyglucan mit einem Verzweigungsgrad von größer Null und maximal 8 % und einer Mischung aus einem Verzweigungen enthaltenden Polyglucan und einem linearen Polysaccharid, wobei der Anteil an Verzweigungen enthaltenden Polyglucan in der Mischung maximal 30 Gew.-%, bezogen auf das Gesamtgewicht des Polysaccharids und Polyglucans, ist.

Der erfindungsgemäße Ersatz zumindest eines Teils der linearen Ausgangsverbindungen durch Verzweigungen enthaltende Polyglucane bedeutet eine wesentliche Vereinfachung und Verbilligung des in Anmeldung Nr. 19737481.6 beschriebenen Verfahrens. Insbesondere vereinfacht das erfindungsgemäße Verfahren den Röckgriff auf natürliche Polyglucanquellen, wodurch eine billige und nachwachsende Rohstoffquelle für das vorliegende Verfahren nutzbar gemacht wird.

In diesem Sinne versteht sich die vorliegende Erfindung als vorteilhafte erfinderische Weiterbildung der vorstehend genannten deutschen Patentanmeldung Nr. 19737481,6.

Weiter betrifft die vorliegende Anmeldung mit dem erfindungsgemäßen Verfahren erhältliche sphärische Mikropartikel.

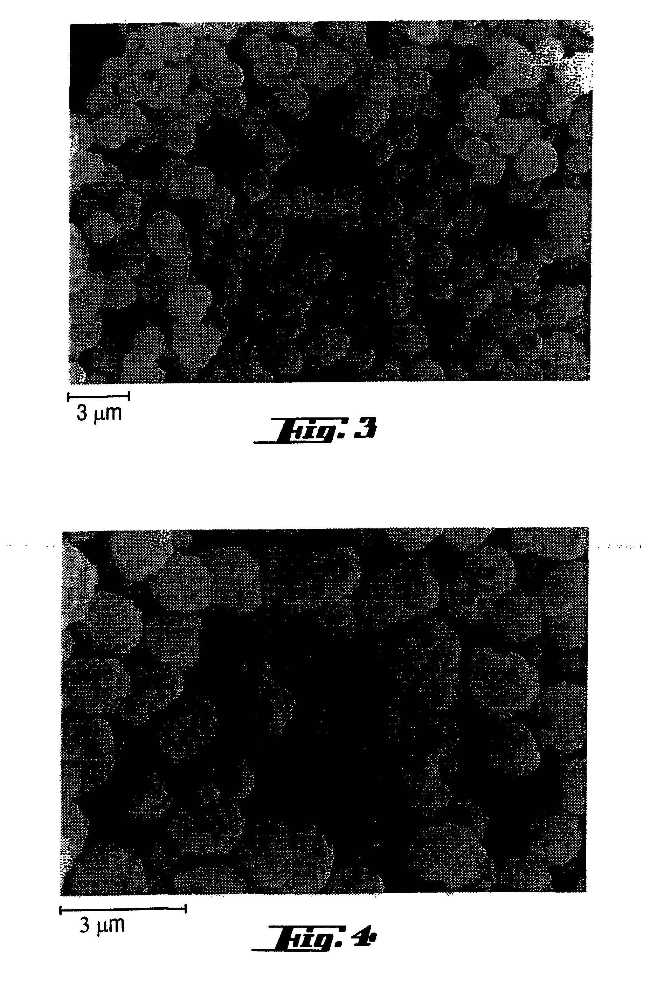

Die Abbildungen 1 bis 4 zeigen rasterelektronenmikroskopische Aufnahmen (REM, Camscan S-4) von erfindungsgemäßen Mikropartikeln:
Abbildung 1: erfindungsgemäße Partikel gemäß Beispiel 1b in 5000 x Vergrößerung,
Abbildung 2: Partikel nach Beispiel 1b in 20000 x Vergrößerung,
Abbildung 3: erfindungsgemäße Partikel nach Beispiel 2 in 5000 x Vergrößerung,
Abbildung 4: erfindungsgemäße Partikel nach Abbildung 3 in 20000 x Vergrößerung,

Polyglucane setzen sich aus Glucanen als monomeren Wiederholungseinheiten zusammen, die über Glykosidbindungen miteinander verknüpft sind. Linearität liegt vor, wenn jedes Monomer in dem Polymergerüst lediglich mit zwei weiteren Wiederholungseinheiten im Molekül über glykosidische Bindung verknüpft ist. Davon ausgenommen sind die beiden Wiederholungseinheiten, die den Anfang und das Ende des Polymers bilden.

Bei drei oder mehr Verknüpfungen pro Wiederholungseinhelt wird von Verzweigung gesprochen. Dabei ergibt sich aus der Anzahl der Hydroxylgruppen pro 100 Wiederholungseinheiten, die nicht am Aufbau des linearen Polymerrückgrats betelligt sind und Verzweigungen ausbilden, der sogenannte Verzweigungsgrad VG. Abgesehen von den beiden an der Ausbildung des linearen Polymerrückgrats beteiligten Hydroxylgruppen, besitzt jede Wiederholungseinheit im Fall von Polyglucan drei freie Hydroxylgruppen.

Für die vorliegende Beschreibung wird für den Ausdruck "Verzweigungen enthaltendes Polyglucan" synonym auch die Bezeichnung "Polyglucan mit Verzweigungen" verwendet.

Gemäß einer ersten Ausführungsform wird anstelle des linearen Polysaccharids ein Polyglucan mit Verzweigungen eingesetzt.

Das Polyglucan weist hierbei einen Verzweigungsgrad von größer 0 bis maximal 8 %, vorzugsweise maximal 5 %, auf.

Bevorzugte Werte für die untere Grenze des Verzweigungsgrades sind wie folgt in 6-Position größer 0,5 % und/oder in jeder anderen Position jeweils größer 0,5 % und insbesondere größer 1 %.

Selbstverständlich können in dieser ersten Ausführungsform dem Polyglucanen mit Verzweigungen beliebige Anteile an wasserunlöslichem linearen Polysaccharid zugemischt werden.

Je höher der Anteil an linearer Struktur ist, umso gleichförmiger sind im Allgemeinen die erhaltenen Mikropartikel. Besonders gleichförmige Partikel können erhalten werden, wenn der Anteil an Verzweigungen enthaltenden Polyglucan maximal 30 Gew.-%, vorzugsweise maximal 20 Gew.-% und insbesondere maximal 10 Gew.-%, beträgt.

In einer zweiten Ausführungsform wird für das erfindungsgemäße Verfahren eine Mischung aus einem wasserunlöslichen linearen Polysaccharid und einem Verzweigungen enthaltenden Polyglucan eingesetzt, wobei der Anteil an Verzweigungen enthaltenden Polyglucan bezogen auf die Gesamtmenge an linearem Polysaccharid und Polyglucan mit Verzweigungen maximal 30 Gew.-%, vorzugsweise 20 Gew.-% und insbesondere 10 Gew.-%, beträgt.
In diesem Fall ist der Verzweigungsgrad des Polyglucans mit Verzweigungen unerheblich.

Gemäß einer bevorzugten Ausführungsform wird in dem erfindungsgemäßen Verfahren mehr als ein Verzweigungen enthaltendes Polyglucan eingesetzt.

Auch hier gilt, daß im allgemeinen die Gleichförmigkeit der Partikel zunimmt, je höher der Anteil an linearer Struktur bzw. je niedriger der Verzweigungsgrad ist.

Die hier verwendeten Polyglucane können Verknüpfungen und Verzweigungen in beliebiger Position der Glucanmonomere aufweisen.
Bevorzugt sind jedoch Polyglucane, deren Polymerrückgrat über 1,4-alphaglykosidische Verknüpfungen gebildet ist. Die Verknüpfung der Verzwelgungen kann beliebig sein.

Gemäß einer bevorzugten Ausführungsform stammen die für die Erfindung verwendeten Polyglucane mit Verzweigungen von Stärke oder Stärkeanaloga ab, die vorzugsweise pflanzlichen oder tierischen Ursprungs sind.

Eine Gruppe von Stärken, die im Rahmen der Erfindung zum Einsatz gelangen können, umfaßt die aus pflanzlichen Rchstoffen gewonnenen Stärken. Hierzu zählen unter anderem Stärken aus Knollen, wie Kartoffeln, Maniok, Maranta, Batata, aus Samen wie Weizen, Mais, Roggen, Reis, Gerste, Hirse, Hafer, Sorghum, aus Früchten, wie Kastanien, Eichein, Bohnen, Erbsen, und ähnlichen Hülsenfrüchten, Bananen, sowie aus Pflanzenmark, z.B. der Sagopalme.

Üblicherweise bestehen die aus pflanzlichen Rohstoffen gewinnbaren Stärken im wesentlichen aus Amylose, einem Poly(1,4-alpha-D-glucan), und Amylopektin, einem Poly(1,4-alpha-D-glucan) mit 1,6-Verzweigungen, in wechseinden Mengenverhältnissen.

Die erfindungsgemäß eingesetzten Polyglucane können auch aus gentechnisch oder biotechnisch veränderten Pflanzen gewonnen werden.

Beispielsweise kann durch die gentechnische oder biotechnische Modifikation die Produktion eines Polyglucans mit höherem linearen Anteil oder eine leichtere Separierung der enthaltenden Stärke bewirkt werden.

Unter Stärkeanaloga werden Verbindungen verstanden, die aus Polyglucanen bestehen, aber nicht-pflanzlichen Ursprungs sind. Ein Beispiel hierfür ist das Glykogen, ein dem Amylopektin entsprechendes Polyglucan, das tierischen Ursprungs ist, oder Dextran, das aus Bakterien gewonnen wird.

Es versteht sich, daß die erfindungsgemäß eingesetzten, Verzweigungen enthaltenden Polyglucane auch biotechnisch, z.B. biokatalytisch oder fermentativ, gewonnen sein können.

Es können auch modifizierte Polyglucane eingesetzt werden, wobei die Polyglucane beispielsweise durch Veresterung und/oder Veretherung einer nicht an der Bildung des Polymerrückgrats betelligten Hydroxylgruppe chemisch modifiziert worden sind. Maßnahmen für derartige Modifizierungen sind dem Fachmann hinlänglich bekannt.

Geeignet sind auch sogenannte alpha-amylase resistente Polyglucane.

Bei Bedarf können diese Stärke und Stärkeanaloga zur Anreicherung der linearen Struktur mit einem beliebigen dafür geeignetem Verfahren aufgereinigt oder behandelt werden.

Geeignete Aufreinigungsverfahren umfassen z. B. Trennprozesse wie Absorptionsverfahren, Fällprozesse mit oder ohne weitere Hilfsmittel, Zentrifugation, Ausnutzen unterschiedlicher Löslichkeiten, oder chromatographische Verfahren.

Es können auch Entzweigungstechniken zum Einsatz kommen, wobei Verzweigungen in dem Polyglucan chemisch oder enzymatisch verringert werden.

Beispielsweise lassen sich mit Enzymen wie Amylasen, iso-Amylasen, Pullulanasen oder Gluconohydrolasen Verzweigungen vorn Polymerrückgrat abspalten, so daß nach ihrer Abtrennung Polymere mit dem gewünschten geringeren Verzweigungsgrad vorliegen.

Weiter kann eine Erhähung der Linearität dadurch erfolgen, daß enzymatisch einzeine Ketten von verzweigten Polyglucanen verlängert werden, wodurch der Verzweigungsgrad verringert wird.

Die erfindungsgemäß einsetzbaren linearen Polysaccharide können beliebigen Ursprungs sein. Sie können aus natürlichen Quellen, die gegebenenfalls gentechnisch oder biotechnisch modifiziert sein können, oder über biotechnische Verfahren gewonnen sein. Eine sehr vorteilhalte Methode für die biotechnische Gewinnung ist beispielsweise in der WO 95/31 553 beschrieben.

Sie können, wie vorstehend für die verzweigten Polyglucane beschrieben, chemisch modifiziert sein.
Es können auch alpha-amylase resistente lineare Polysaccharide eingesetzt werden, wie sie z.B. in der prioritätsälteren nicht-vorveröffentlichten deutschen Patentanmeldung Nr. 198 30 618.0.

Insbesondere können dieselben linearen Polysaccharide eingesetzt werden wie sie in der deutschen Anmeldung Nr. 19737481,6 beschrieben sind.

Bevorzugte Beispiele sind lineare Polyglucane wie Poly(1,4-alpha-D-glucan) und Poly(1,3-beta-D-glucan), wobei Poly(1,4-alpha-D-glucan), insbesondere biotechnisch gewonnen, besonders bevorzugt ist.

Im folgenden wird soweit auf die wasserunlöslichen Polyglucane mit Verzweigungen und die wasserunlöslichen linearen Polysaccharide gemeinsam bezug genommen wird, der Ausdruck "Polyglucan/saccharid" verwendet.

Unter dem Begriff "wasserunlösliche Polyglucane/saccharide" werden für die vorliegende Erfindung Verbindungen verstanden, die nach der Definition des Deutschen Arzneibuches (DAB = Deutsches Arzneibuch)

Wissenschaftliche Verlagsgesellschaft mbH, Stuttgart, Govi-Verlag GmbH, Frankfurt, 9. Auflage, 1987) entsprechend den Klassen 4 bis 7 unter die Kategorien "wenig löslich", "schwer lösliche", "sehr schwer lösliche" bzw. "praktisch unlösliche" Verbindungen fallen.

Für die vorliegende Erfindung sind schwer lösliche bis praktisch unlösliche Verbindungen, insbesondere sehr schwer lösliche bis praktisch unlösliche Verbindungen, bevorzugt.

Im Fall der erfindungsgemäß verwendeten Polyglucane/saccharide bedeutet dies, daß vorzugsweise mindestens 98 % der eingesetzten Menge, insbesondere mindestens 99,5 %, unter Normalbedingungen ( T = 25 °C +/- 20 %, p= 101325 Pascal +/- 20 %) in Wasser unlöslich ist ( entsprechend den Klassen 4 bzw. 5).

"Sehr schwer löslich" entsprechend Klasse 6 kann durch folgende Versuchsbeschreibung veranschaulicht werden:
Ein Gramm des zu untersuchenden Polyglucans/saccharids werden in 1 l entionisierten Wasser auf 130 °C unter einem Druck von 1 bar erhitzt. Die entstehende Lösung bleibt nur kurzzeitig über wenige Minuten stabil. Beim Erkalten unter Normalbedingungen fällt die Substanz wieder aus. Nach Abkühlung auf Raumtemperatur und Abtrennung mittels Zentrifugieren können unter Berücksichtigung der experimentellen Verluste mindestens 66 % der eingesetzten Menge zurückgewonnen werden.

Die Molekulargewichte M_{w} (Gewichtsmittel, bestimmt mittels Gelpermeationschromatographie im Vergleich zu einer Eichung mit Pullulanstandard) der erfindungsgemäß verwendeten Polyglucane/saccharide können in einem weiten Bereich von 10³ g/mol bis 10⁷ g/mol variieren. Bevorzugt liegt das Molekulargewicht M_{w} in einem Bereich von 10⁴ g/mol bis 10⁵ g/mol und besonders bevorzugt von 2 x 10⁴ g/mol bis 5 x 10⁴ g/mol. Ein weiterer vorteilhafter Bereich ist von 2 x 10³ bis 8 x 10³ g.mol⁻¹.

Es hat sich gezeigt, daß die Molekulargewichte für das Verzweigungen enthaltende Polyglucan auch höher liegen kann.

Die Molekulargewichtsverteilung bzw. Polydispersität M_{w}/Mₙ kann ebenfalls in weiten Bereichen je nach Ursprung und Herstellungsverfahren des Polyglucans/saccharids varieren. Bevorzugte Werte sind von 1,01 bis 50, insbesondere von 1,5 bis 15. Dabei nimmt die Polydispersität mit einer bimodalen Verteilung der Molekulargewichte zu.

Wie bereits erwähnt, können erfindungsgemäß den Polyglucanen mit Verzweigungen wasserunlösliche lineare Polysaccharide, vorzugsweise wasserunlösliche lineare Polyglucane, beigemischt werden.

Es können auch andere Polymere, insbesondere andere biokompatible oder bioabbaubare Polymere, zugesetzt werden. Dabei hängt die Menge des oder der anderen Polymeren, die zugesetzt werden, ohne daß die sphärische Gestalt und/oder sonstige Eigenschaften der herzustellenden Mikropartikel verändert werden, stets von dem zugesetzten Polymer ab. Sie kann bis zu 10 % oder mehr betragen, bezogen auf den Anteil Polyglucan mit Verzweigungen und ggf. lineares Polysaccharid, in bestimmten Fällen auch weniger. Die zulässige maximale Menge hängt von dem jeweiligen Einzelfall ab und kann von einem Fachmann leicht durch Routineversuche bestimmt werden.

Zur Herstellung der erfindungsgemäßen Mikropartikel werden die Ausgangssubstanzen, wie z. B. das Polyglucan mit Verzweigungen und ggf. lineare Polysaccharid, in einem Lösungsmittel gelöst. Beispiele für geeignete Lösungsmittel sind Dimethylsulfoxid (DMSO), Formamid, Acetamid, N,N-Dimethylformamid, N,N-Dimethylacetamid, N-Methylmorpholin-N-oxid in Gegenwart von Wasser, weitere N-substituierte Morpholin-N-oxide, wäßrige Lösungen mit hohem oder niedrigem pH-Wert oder Mischungen aus den vorstehend genannten Lösungsmitteln, wobei DMSO besonders bevorzugt ist. Selbstverständlich können auch andere, dem Fachmann für diesen Zweck geläufige Lösungsmittel verwendet werden.

Die Gesamtkonzentration (Konzentration Polyglucan mit Verzweigungen plus ggf. Konzentration an linearem Polysaccharid) in dem Lösungsmittel kann je nach Bedarf in weiten Grenzen variieren. Vorzugsweise liegt sie In einem Bereich von 0,02 g (Polyglucan mit Verzweigungen+ Polysaccharid)/ml (Lösungsmittel) bis 1,0 g/ml, insbesondere von 0,05 g/ml bis 0,8 g/ml und besonders bevorzugt von 0,3 g/ml bis 0,6 g/ml.

Beispiele für Fällmittel sind Wassar, Dichlormethan, ein Gemisch aus Wasser und Dichlormethan, Gemische aus Wasser und Alkoholen wie Methanol, Ethanol, Isopropanol, wobei Wasser sowie ein Gemisch aus Wasser und Dichlormethan besonders bevorzugt sind.

Gemäß einer bevorzugten Ausführungsform wird als Fällmittel in dem erfindungsgemäßen Verfahren Wasser oder ein wässriges Medium eingesetzt.

Vorzugsweise wird das Verhältnis Lösungsmittel zu Fällmittel in einem Bereich von 1 : 1000 bis 1 : 4 (Tell Lösungsmittel / Teile Fälimittel), vorzugsweise 1 : 100 bis 1 : 10 und insbesondere 1 : 70 bis 1 : 30 ausgewählt.

Gemäß einer bevorzugten Ausführungsform werden die die Ausgangssubstanzen enthaltene Lösung und das Fällmittel bei einer Temperatur zwischen 20 °C und 50 °C zusammengebracht.

Erfolgt das Zusammenmischen bei erhöhter Temperatur, kann das entstehende Gemisch anschließend bei Bedarf gekühlt werden.

Dabei ist es unerheblich, in welcher Reihenfolge das Lösungsmittel und das Fällmittel zusammengebracht werden, z.B. ob das Fällmittel zum Lösungsmittel oder umgekehrt gegeben wird.
Wichtig ist jedoch, daß eine schnelle Durchmischung gewährleistet wird.

Die Temperatur während des Fällprozesses wird im allgemeinen bei einem Wert von plus 10 °C bis minus 10 °C, vorzugsweise plus 5 °C und minus 5 °C, gehalten. Bei Bedarf kann sie auch höher oder niedriger gewählt werden.

Der Fällprozeß kann relativ langsam bei tiefer Temperatur über Nacht durchgeführt werden.
Er kann durch Variation der Temperatur und des Fällmittels beeinflußt und gesteuert werden.

Weiter können die Prozeßführung sowie die Eigenschaften der Mikropartikel, wie Größe durch Zusatz von Fällungshilfsmitteln beeinflußt werden.

Falls gekühlt wird, muß sichergestellt sein, daß das Gemisch aus Lösungsmittel und Fällmittel liquide bleibt und nicht erstarrt.

Geeignete Fällungshilfsmittel sind z.B. oberflächenaktive Stoffe wie Natriumdodecylsulfat, N-Methylgluconamid, Polysorbate (z.B. Tween (eingetragene-Marke)), Alkylpolyglycolether, Ethylenoxid-Propylenoxid-Blockpolymere (z.B. Pluronic (eingetragene Marke)), Alkylpolyglycolethersulfate, generell Alkylsulfate und Fettsäureglycolester, Zucker wie z.B. Fructose, Saccharose, Glucose und wasserlösliche Cellulosederivate.

Die oberflächenaktiven Stoffe können anionischer, kationischer oder nicht-ionischer Natur sein.

Prinzipiell kann jedes wasserlösliche Cellulosederivat verwendet werden, sofern es als Fällungshilfsmittel geeignet ist.
Es kann sich hierbei um chemisch modifizierte Cellulosen jedweder Art handeln. Beispiele sind Celluloseester und Celluloseether und deren Mischformen. Konkrete Vertreter sind z.B., Hydroxypropylmethylcellulosen, Hydroxyethylcellulosen, Carboxymethylcellulosen, Celluloseacetate, Cellulosebutyrate, Cellulosepropionate, Celluloseacetobutyrate, Celluloseacetopropionate, Cellulosenitrate, Ethylcellulosen, Benzylcellulosen, Methylcellulosen.

Es können auch Mischungen von verschiedenen wasserlöslichen Cellulosederivaten eingesetzt werden.

Unter dem Begriff "wasserlösliche Cellulosederivate" werden für die vorliegende Erfindung Verbindungen verstanden, die nach der Definition des Deutschen Arzneibuches (DAB = Deutsches Arzneibuch) Wissenschaftliche Verlagsgesellschaft mbH, Stuttgart, Govi-Verlag GmbH, Frankfurt, 9. Auflage, 1987) unter die Katagorie sehr leicht löslich bis schwer löslich fallen.

Üblicherweise werden die Hilfsmittel dem Fällmittel zugesetzt. Die verwendete Mange hangt von dem jeweiligen Einzelfall sowie den erwünschten Partikeleigenschaften ab, wobei die Bestimmung der jeweils vorteilhaften Menge dem Fachmann geläufig ist.

Als vorteilhaft haben sich Konzentrationen von 2 g (Hilfsmittel)/l (Fällmittel) bis 150 g/l, vorzugsweise von 5 g/l bis 80 g/l und insbesondere 8 g/l bis 20 g/l, erwiesen. Dies gilt insbesondere auch für das wasserlösliche Cellulosederivat.

Interessanterweise hat sich gezeigt, daß der Anteil an besonders kleinen Partikeln gesteigert werden kann, wenn dem Fällmittel heißwasserlösliches Poly-alpha-Dglucan zugesetzt wird.

Es können hierfür dieselben Poly-alpha-D-glucanverbindungen eingesetzt werden wie sie im Zusammenhang mit den Polyglucanen mit Verzweigungen bzw. den linearen Polysacchariden genannt worden sind.

Bevorzugte Beispiele sind native oder chemisch modifizierte Stärken, aus diesen Stärken gewonnene Poly-alpha-D-glucane sowie stärkeanaloge Verbindungen.

Unter stärkeanaloge Verbindungen werden Verbindungen verstanden, die aus Poly-alpha-D-glucanean bestehen, aber nicht-pflanzlichen Ursprungs sind. Ein Beispiel hierfür ist Glykogen oder Dextran.

Die heißwasserlöslichen Poly-alpha-D-glucane können als Mischung aus einem linearen und einem verzweigten Anteil eingesetzt werden, wie sie z.B. in Stärke vorliegt. In diesem Fall sollte der Anteil an linearem Poly-alpha-D-glucan mehr als 15 Gew.-%, vorzugsweise 50 bis 99,5 Gew.-%, insbesondere 60 bis 90 Gew.-% und ganz besonders bevorzugt 65 bis 80 Gew.-%, bezogen auf die Gesamtmenge Poly-alpha-D-glucan im Fällmittel, betragen.

Sie können aber auch aus verzweigten Strukturen bestehen, wie sie z.B. im Amylopektin oder im Glykogen vorliegen.

Im Rahmen der vorliegenden Erfindung bedeutet "heißwasserlöslich", daß die Poly-alpha-D-glucane bei Raumtemperarur im wesentlich unlöslich sind, wobei vorzugsweise der gleiche Maßstab wie für den Begriff "wasserunlöslich" in Zusammenhang mit den verzweigten Polyglucanen bzw. linearen Polysacchariden gilt.
Unter dem Begriff "Lösung" bzw. "Löslichkeit" werden insbesondere auch Suspensionen bzw. die Ausbildung von Suspensionen verstanden wie sie bei der Lösung von Stärke auftreten.

Beispielsweise zeigen die erfindungsgemäß bevorzugten heißwasserlöslichen Stärken bei Raumtemperatur so gut wie keine Löslichkeit in Wasser, während die sogenannten kaltwasserlöslichen Stärken unter diesen Bedingungen leichter löslich sind.

Die heißwasserlöslichen Stärken sind insbesondere dadurch charakterisert, daß sie bei Erhitzen unter Eigendruck, z.B. in einem Autoklaven, auf eine Temperatur im Bereich von etwa 100 bis etwa 160 °C Lösungen bilden, wobei die jeweilige Temperatur von der Art der Stärke abhängt.

Beispielsweise kann Kartoffelstärke bei ca. 100 °C bis zur völligen Auflösung gekocht werden, während Maisstärke eine Temperatur von ca. 125 °C erfordert.

Für das erfindungsgemäße Verfahren werden die heißwasserlösllchen Poly-alpha-D-glucans dem Fällmittel vorzugsweise in maximaler Konzentration zugesetzt, d.h. es wird eine gesättigte Lösung hergestellt.

Weitere geeignete Bereiche sind von mehr als 0,001 Gew.-% bis 10 Gew.-%, bevorzugt von 0,01 bis 2 Gew.-% und insbesondere von 0,05 Gew.-% bis 0,5 Gew.-%, bezogen auf die eingesetzte Menge an Fälimittel.

Aufgrund ihrer Naturidentität sind die meisten der erfindungsgemäß eingesetzten wasserunlöslichen Verzweigungen enthaltenden Polyglucane und ggf. wasserunlöslichen linearen Polysaccharide sowie deren Abbauprodukte biokompatibel und biologisch abbaubar. Sie besitzen eine hohe Gewebeverträglichkeit und reichern sich nicht in einem tierischen, insbesondere menschlichen, Organismus an.
Unter biologischem Abbau wird dabei jedweder in vivo ablaufende Vorgang verstanden, der zu einem Abbau oder einer Zerstörung der Substanzen, hier der wasserunlöslichen Polyglucane/Polysaccharide führt.

Diese Eigenschaften der Biokompatibilität und der biologischen Abbaubarkeit sind insbesondere für Verwendungen vorteilhaft, die einen menschlichen oder tierischen Organismus betreffen, z. B. in der Medizin, Pharmazie oder Kosmetik.

Die nach dem erfindungsgemäßen Verfahren erhältlichen sphärischen Mikropartikel, die ebenfalls Gegenstand dieser Erfindung sind, weisen ebenso wie die in der deutschen Patentanmeldung Nr. 19737481.6 beschriebenen Mikropartikel eine regelmäßige sphärische Gestalt, enge Größenvertellung und gute mechanische Eigenschaften auf.

Gemäß einer bevorzugen Ausführungsform bestehend die erfindungsgemäßen sphärischen Mikropartikel ganz oder teilweise aus mindestens einem wasserunlöslichen Verzweigungen enthaltenden Polyglucan. Vorzugsweise beträgt der Verzweigungsgrad des Verzweigungen enthaltenden Polyglucan mehr als 0 % und ist maximal 8 %.

Dabei ist es bevorzugt, dass diese sphärischen Mikropartikel zusätzlich mindestens ein wasserunlösliches lineares Polysaccharid enthalten. Besonders bevorzugt ist es hierbei, dass das Verzweigungen enthaltende Polyglucan in den erfindungsgemäßen sphärischen Mikropartikeln verzweigtes Poly (1,4-α-D-Glucan) ist. Ebenso ist das wasserunlösliche lineare Polysaccharid in den erfindungsgemäßen sphärischen Mikropartikeln bevorzugt lineares Poly (1,4-α-D-Glucan).

Die Partikel können mittlere Durchmesser (Zahlenmittelwert) aufweisen von 1 nm bis 100 µm, bevorzugt 100 nm bis 10 µm und besonders bevorzugt 1 µm bis 5 µm.

Sphärisch im Sinne der Erfindung bedeutet, daß die Mikropartikel annähernd Kugelform besitzen. Bei Beschreibung einer Kugel durch von einem gemeinsamen Ursprung ausgehende, in den Raum gerichtete Achsen gleicher Länge, die den Radius der Kugel in allen Raumrichtungen definieren, ist für die sphärischen Mikropartikel eine Abweichung der der Achsenlängen vom idealzustand der Kugel von 1 % bis 40 % möglich. Bevorzugt werden sphärische Mikropartikel mit Abweichungen bis 25 %, besonders bevorzugt bis 15 % erhalten.

Die Oberfläche der sphärischen Mikropartikel kann makroskopisch mit einer Himbeere verglichen werden, wobei die Tiefe von Unregelmäßigkeiten auf der Partikeloberfläche, wie Eindellungen oder Einschnitte, maximal 20 % des mittleren Durchmessers der sphärischen Mikropartikel beträgt.

Weiter zeigen die erfindungsgemäßen Mikropartikel vorzugsweise eine Dispersität D = Gewichtsmittelwert des Durchmessers (d_{w})/Zahlenmittelwert des Durchmessere (dₙ) von 1,0 bis 10,0, vorzugsweise von 1,5 bis 8,0 und insbesondere von 2,0 bis 4,0.

Die hier benutzten Mittelwerte sind wie folgt definiert:
dₙ = Summe nₗ x dₗ / Summe nₗ = Zahlenmittelwert
dₙ = Summe nₗ x dₗ² / Summe nₗ x dₗ = Gewichtsmittelwert
nₗ = Anzahl der Partikel mit Durchmesser dₗ,

So können sie in reiner Form oder als Wirkstoffträger auch im weitestem Sinne eingesetzt werden, beispielsweise
- als Additive in der Kosmetik für Salben, Puder, Cremes oder Pasten.
- als Träger für Wirksubstanzen in pharmazeutischen, tiermedizinischen und anderen,
- als Glättungsmittel z.B. zum Verschließen von Poren oder Glätten von Graten,
- als Lebensmittelzusatzstoff, z.B. als Füllkomponente oder zum Verbessern von rheologischen Eigenschaften,
- als Additiv zur Veredlung von z.B. Emulsionspolymerisaten,
- als Trennhilfen, z.B. für die Abtrennung von Verunreinigungen,
- als Verkapselungsmaterial,
- als Träger für magnetische Partikel,
- als Füllmittel für Insbesondere bioabbaubare Polymere oder technische Polymere z.B. zur Eigenschaftskontrolle,
- als Additiv zur Eigenschaftskontrolle, z.B. der Porosität, des Gewichts oder der Farbe,
- als Partikelstandard zur Eichung oder Bestimmung der Partikelgröße unbekannter Materialien,
- als Trägermaterial zur kontrollierten, z.B. retardierten Wirkstoffabgabe,
- als Füllstoff zur Eigenschaftsverbesserung von technischen oder biokompatiblen Polymeren, und
- in diagnostischen Tests, z. B. als Ultraschallmittel.

Im folgenden wird die Erfindung anhand von Beispielen näher erläutert. Diese Beispiele dienen der Veranschaulichung und haben keine limitierende Bedeutung.

### Beispiel 1 - Vergleichsbeispiel

### Herstellung von Mikropartikeln mit einer amyloseangereicherten Stärke

a. Anreicherung des linearen Anteils (Amyloseanreicherung):
   30 g Stärke (Hylon® VII, Marke der Fa. National Starch and Chennical company ) wurden in 500 ml Dimethylsulfoxid (DMSO, Riedel de Haen) 24 Stunden (h) bei Im folgenden wird die Erfindung anhand von Beispielen näher erläutert. Diese Beispiele dienen der Veranschaulichung und haben keine limitierende Bedeutung.

### Beispiel 1

### Herstellung von Mikropartikeln mit einer amyloseangereicherten Stärke

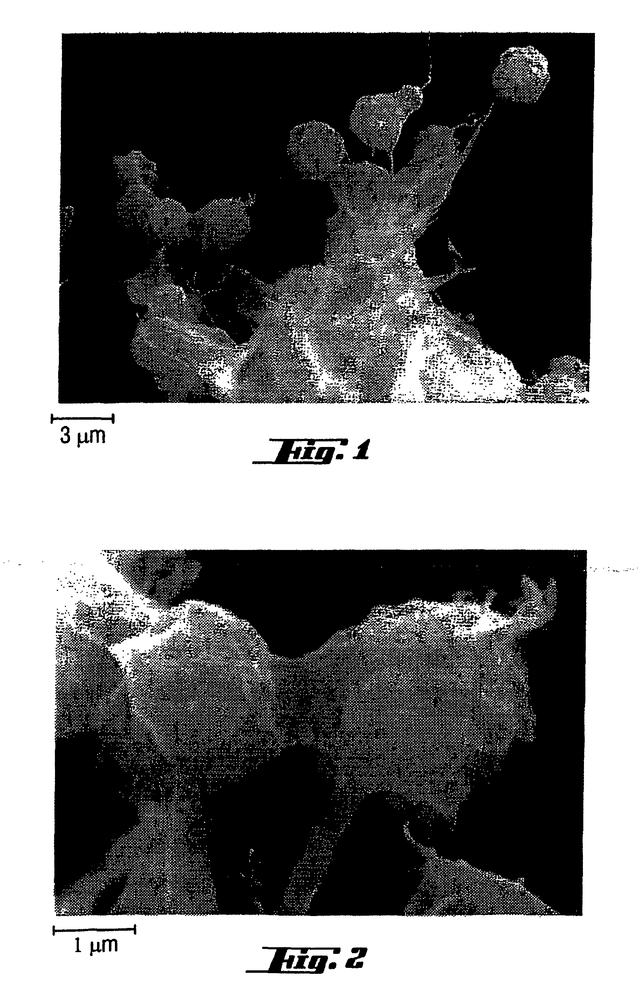

a. Anreicherung des linearen Anteils (Amyloseaneicherung):
   30 g Stärke (Hylon VII®, Marke der Fa. (National Starch and Chemical Company, Maisstärke mit etwa 70 % Amylose-Anteil) wurden in 600 ml Dimethyl sulfoxid (DMSO. Riedel de Haen) 24 Stunden (h) bei Reumtemperatur gerührt. Es bildete sich eine trübe Mischung. Es wurde 20 min. bei 4000 Umdrehungen/Minute (U/min.) abzentrifugiert (Labofuge GI. der Fa. Heraaus). Die erhaltene Lösung wurde in 1 l 1-Butanol gefält und etwa 1 h stehengelassen. Anschließend wurde filtriert. Der Filterkuchen wurde in 3l kochendem Wasser gelöst. Es wurde auf 60°C gekühlt und 3 g Thymol (Fa. Fluka) zugegeben. Der Ansatz wurde 3 Tage stehengelassen. Danach wurde er für 46 min. aufgekocht. Nach dem Abkühlen wurde anschlieflend 1 l 1-Butanol dazugegeben und über Nacht ausgefällt. Es wurde 20 min. bei 4000 V/min. zentrifugiert. Der erhaltene Rückstand wurde gefriergetrocknet (Gefriertrocknung der Fa. Christ).
   Ausbeute: 11,7 g (39 %).
b. Verwendung der amyloseangereicherten Stärke zur Herstellung von Mikropartikeln:
   1,0 g der vorstehend erhaltenen amylosaangereicherten Stärke wurde in 5 ml DMSO (Riedel de Haen) bei 60 °C innerhalb weniger Minuten gelöst. Die Lösung wurde unter Rühren innerhalb weniger Sekunden in 100 ml Wasser eingetropft. Der erhaltene Ansatz wurde 16 h bei 5 °C stahengelassen. Es bildete sich ein feiner weißer Niederschlag in einer milchigen Suspension aus. Der Niederschlag, der die Partikel enthielt, wurde abgetrennt, indem der gesamte Ansatz homogen suspendiert und anschließend 10 min bei 3000 U/min. zentrifugiert wurde (Labofuge GL der Fa. Heraeus). Der erhaltene feste Rückstand wurde insgesamt dreimal mit bidestiliertem Wasser aufgeschlämmt und unter den gleichen Bedingungen wie zuvor zentrifugiert. Anschließend wurde der erhaltene Feststoff in knapp 10 ml bidestilliertem Wasser aufgeschlämmt, eingefroren und lyophilislert (Gefriertrocknung: Christ Delta 1-24 KD).
   Zur Charakterisierung der Partikel wurden Rasterelektronenmikroskopaufnahmen angefertigt (REM, Camscan S-4), die in Abbildungen 1 und 2 gezeigt sind.
   Anhand der Aufnahmen konnte der Anteil an Partikeln auf etwa 30 % geschätzt werden.
c. Nach Wiederholung des unter a beschriebenen Anreicherungsverfahrens erhöhte sich der Partikelanteil auf etwa 56 %, nach einer dritten Wiederholung auf etwa 70 %.

### Beispiel 2

### Herstellung unter Verwendung einer Mischung aus verzweigtem und linearem Poly(1,4-alpha-D-glucan):

Der Fällungsprozeß wurde im wesentlichen wie unter Beispiel 1b beschrieben durchgeführt.
Als Ausgangssubstanz wurde eine Mischung aus 98 mg Poly(1,4-alpha-D-glucan) (98 %) und 2 mg Glykogen (2 %) aus Austem (Fa. Fluka) verwendet.
Ausbeute 65,9 mg (60 %).
Aus REM-Aufnahmen ergibt sich ein Anteil an Partikeln von mehr als 95 % (Abbildungen 4 und 5).

### Beispiel 3

### Herstellung unter Verwendung einer Mischung aus verzweigtem und linearem Poly(1,4-alpha-D-glucan):

Der Fällungsprozeß wurde im wesentlichen wie unter Beispiel 1b beschrieben durchgeführt.
Als Ausgangssubstanz wurde eine Mischung aus 90 mg Poly(1,4-alpha-D-glucan) (90 %) und 10 mg Amylopektin (10 %) (Amioca Powder der Fa. National Starch) verwandet.
Aus den REM-Aufnahmen ergibt sich ein Anteil an Partikeln von mehr als 85 %.

### Beispiel 4

a. Enzymatische Entzweigung von Amylopektin zur Anreicherung der linearen Strukturein.
   In einem 4-Hals-Rührer mit Rückflußkühler (Polymerapparatur) wurden 200 ml entionisiertes Wasser vorgelagt. Dazu wurden 50 g Amylopektin (Amioca Powder TFL der Fa. National Starch) gegeben. Es wurde unter Rühren auf 125 °C erhitzt und der Ansatz 1h bei dieser Temperatur gehalten, wobei die Viskosität zur ahm. Anschließend wurde im Autoklaven unter Eigendruck 30 min. auf 130 °C erhitzt, wobei eine klare, homogen wirkende, dickflüssige Lösung erhalten wurde.
   Es wurde auf 58 °C abgekühlt und 60 mg Pullulanase aus Bacillus sp. (1,62 U/mg) (Fa. Fluks), die vorher in 1ml entionisiertem Wasser gelöst worden waren, dazugegeben. Der die Pululanase enthaltende Ansatz wurde 12 h bei 58 °C gerührt, wobei die Massa dünnflüssig wurde. Danach wurde auf Raumtemperatur abgekühlt, wobei die Lösung eine weißliche Färbung annahm.
   Nach 6 h Stunden wurde eine Probe (Probe 1) entnommen und aufgearbeitet. Der verbleibande Rückstand wurde nach 12 h abgetrennt und gefriergetrocknet (Probe 2).
   Ausbeute: 48,5 g.
b. Verwendung des unter a. erhaltenen enzymatisch aufgearbeiteten Amylopaktine zur Herstellung von Mikropartikeln
   Es wurde im wesentlichen das gleiche Verfähren wie in Beispiel 1b. angewandt.

Im Ergebnis zeigte sich, daß mit Zunahme der linearen Strukturen der Anteil an ausgebildeten Partikeln zunimmt:
Probe 1: etwa 50 % Partikel
Probe 2: etwa 80 % Partikel.

### Beispiel 5

### Bestimmung der Löslichkeit von Polysacchariden und Klassifizierung nach dem Deutschen Arzneibuch (DAB)

564 mg Poly(1,4-alpha-D-glucan) wurden in etwa 0,5l bidestillierten Wasser bei 1,3 bar und 130 °C 1,5 Stunden in einem Autoklaven (Apparat Certoclav) erhitzt. Vorher war das Gewicht des Autoklaven bestimmt worden.
Anschließend wurde die Apparatur entspannt und bei Raumtemperatur abgekühlt. Der Inhalt wurde gewogen. Er entsprach 501,74 g.
Nach weiteren 24 Stunden wurde zentrifugiert und dekantiert. Der erhaltene feste Rückstand wurde getrocknet und ausgewogen. Es wurden 468 mg erhalten, woraus sich ein gelöster Anteil von 96 mg errechnet.

Bezogen auf die eingesetzte Menge an Lösungsmittel folgt daraus, daß zur Lösung von 1 mg Poly(1,4-alpha-D-glucan)) 5226 mg Wasser erforderlich sind. Nach der Klassifizierung gemäß DAB fällt diese Substanz somit unter die Klasse "sehr schwer löslich", Gemäß DAB fallen unter diese Klasse alle Substanzen, für die zwischen 1.000 und 10.000
Teile Lösungsmittel erforderlich sind, um 1 Teil der Substanz in Lösung zu bringen.

Dies ist von den 7 Klassen, in die die Löslichkeit gemäß DAB eingeteilt wird, die Klasse 6, wobei die Einteilung von der Klasse 1 "sehr leicht löslich" bis Klasse 7 "praktisch unlöslich" reicht.

## Patentansprüche

1. Verfahren zur Herstellung von sphärischen Mikropartikeln, die ganz oder teilweise aus mindestens einem wasserunlöslichen Polysaccharid bestehen, wobei das mindestens eine wasserunlösliche Polysaccharid, welches den Löslichkeitsklassen 5-7 des Deutschen Arzneibuch (DAB) entspricht, in einem Lösungsmittel oder Lösungsmittelgemisch gelöst wird, die gebildete Lösung in ein Fällmittel oder Fällmittelgemisch eingebracht wird, das dabei entstehende Gemisch gegebenenfalls gekühlt wird und die gebildeten Mikropartikel abgetrennt werden, **dadurch gekennzeichnet, daß** das wasserunlösliche Polysaccharid ausgewählt ist unter einem Verzweigungen enthaltenden Polyglucan mit einem Verzweigungsgrad von 0,5 % bis maximal 8 % und einer Mischung aus einem Verzweigungen enthaltenden Polyglucan und einem linearen Polysaccharid, wobei der Anteil an Verzweigungen enthaltenden Polyglucane in der Mischung maximal 30 Gew.-% bezogen auf das Gesamtgewicht des Polysaccharids und Polyglucans ist.

2. Verfahren nach Anspruch 1, wobei das wasserunlösliches Polysaccharid eine Mischung aus einem Verzweigungen enthaltenden Polyglucan mit einem Verzweigungsgrad von größer Null und maximal 8 % und einem linearen Polysaccharid ist.

3. Verfahren nach Anspruch 1 oder 2, wobei das Verzweigungen enthaltende Polyglucan aus einer pflanzlichen Quelle stammt.

4. Verfahren nach Anspruch 3, wobei die pflanzliche Quelle Stärke ist.

5. Verfahren nach einem der Ansprüche 1 oder 2, wobei das Verzweigungen enthaltende Polyglucan aus einer tierischen Quelle stammt.

6. Verfahren nach Anspruch 5, wobei die tierische Quelle Glykogen ist.

7. Verfahren nach einem der vorhergehenden Ansprüche, wobei das Polymergerüst des Verzweigungen enthaltenden Polyglucans aus D-Glucanmonomeren über 1,4-alpha-glykosidische Verknüpfungen aufgebaut ist.

8. Verfahren nach einem der vorhergehenden Ansprüche, wobei das Verzweigungen enthaltende Polyglucan chemisch modifiziert worden ist.

9. Verfahren nach einem der vorhergehenden Ansprüche, wobei als Verzweigungen enthaltendes Polyglucan ein alpha-amylase resistentes Polyglucan eingesetzt wird.

10. Verfahren nach einem der vorhergehenden Ansprüche, wobei mehr als ein Verzweigungen enthaltendes Polyglucan eingesetzt wird.

11. Verfahren nach einem der vorhergehenden Ansprüche, wobei die Lösung und das Fällmittel bei einer Temperatur im Bereich von 20 bis 50 °C vermengt werden und das entstehende Gemisch auf eine Temperatur im Bereich von plus 10 °C bis minus 10 °C abgekühlt wird.

12. Verfahren nach Anspruch 11, wobei das entstehende Gemisch auf eine Temperatur im Bereich von plus 5 °C bis minus 5 °C abgekühlt wird.

13. Verfahren nach einem der vorhergehenden Ansprüche, wobei als Fällmittel Wasser oder ein wäßriges Medium eingesetzt wird.

14. Verfahren nach einem der vorhergehenden Ansprüche, wobei als Lösungsmittel Dimethylsulfoxid eingesetzt wird.

15. Verfahren nach einem der vorhergehenden Ansprüche, wobei das wasserunlösliche lineare Polysaccharid ein lineares Polyglucan ist.

16. Verfahren nach einem der vorhergehenden Ansprüche, wobei als wasserunlösliches lineares Polysaccharid Poly(1,4-alpha-D-glucan) eingesetzt wird.

17. Verfahren nach einem der vorhergehenden Ansprüche, wobei als wasserunlösliches lineares Polysaccharid Poly(1,3-beta-D-glucan) eingesetzt wird.

18. Verfahren nach einem der vorhergehenden Ansprüche, wobei das wasserunlösliche lineare Polysaccharid ein chemisch modifiziertes Polysaccharid ist.

19. Verfahren nach Anspruch 18, wobei das wasserunlösliche lineare Polysaccharid in mindestens einer der Positionen, die nicht an der Ausbildung der Polymerkette beteiligt ist, vorzugsweise in Position 2-, 3- und/oder 6, verestert und/oder verethert worden ist.

20. Sphärische Mikropartikel mit glatter Oberfläche, d.h. wobei die Oberfläche der Mikropartikel Eindellungen oder Einschnitte aufweist, die maximal 20% des mittleren Durchmessers der Mikropartikel betragen, erhältlich nach einem Verfahren nach einem der Ansprüche 1 bis 19.

21. Sphärische Mikropartikel, bestehend ganz oder teilweise aus mindestens einem wasserunlöslichen Verzweigungen enthaltenden Polyglucan, welches den Löslichkeitsklassen 5-7 des Deutschen Arzneibuch (DAB) entspricht.

22. Sphärische Mikropartikel nach Anspruch 21, wobei der Verzweigungsgrad des Verzweigungen enthaltenden Polyglucans 0,5% bis maximal 8 % ist.

23. Spärische Mikropartikel nach einem der Ansprüche 21 bis 22, zusätzlich enthaltend mindestens ein wasserunlösliches lineares Polysaccharid.

24. Sphärische Mikropartikel nach einem der Ansprüche 21 bis 23, wobei das Verzweigungen enthaltende Polyglucan verzweigtes Poly(1,4-alpha-D-glucan) ist.

25. Sphärische Mikropartikel nach einem der Ansprüche 21 bis 24, wobei das wasserunlösliche lineare Polysaccharid lineares Poly(1,4-alpha-D-glucan) ist.

26. Verwendung von sphärischen Mikropartikeln nach einem der Ansprüche 20 bis 25 zur Trennung von Stoffgemischen.

27. Verwendung von sphärischen Mikropartikeln nach einem der Ansprüche 20 bis 25 als Füllmittel in Polymeren.

28. Verwendung von sphärischen Mikropartikeln nach einem der Ansprüche 20 bis 25 in diagnostischen Test.

## Claims

1. Procedure for the manufacture of spherical micro-particles which consist partially or wholly of at least one water-insoluble polysaccharide, whereby the at least one water-insoluble polysaccharide which corresponds to solubility classes 5 - 7 of the "Deutsches Arzneibuch (DAB)" [German Pharmaceutical Register] is dissolved in a solvent or a mixture of solvents after which the resulting solution is mixed with a precipitating agent or mixture of precipitating agents with the resulting mixture being cooled, if necessary, and the micro-particles thus formed are separated out, **characterised in that** the water-insoluble polysaccharide is selected from a branched polyglucane having a degree of branching between 0.5 and a maximum of 8% and a mixture of a branched polyglucane and a linear polysaccharide, where the proportion of branched polyglucanes in the mixture does not exceed a maximum of 30% by weight expressed in terms of the total weight of the polysaccharide and the polyglucane.

2. Procedure in accordance with claim 1, whereby the water-insoluble polysaccharide is a mixture of a branched polyglucane with a degree of branching greater than zero and up to a maximum of 8% and a linear polysaccharide.

3. Procedure in accordance with claim 1 or 2, whereby the branched polyglucane is of vegetable origin.

4. Procedure in accordance with claim 3, whereby the vegetable origin is starch.

5. Procedure in accordance with one of claims 1 or 2 whereby the branched polyglucane is of animal origin.

6. Procedure in accordance with claim 5, whereby the animal source is glycogen.

7. Procedure in accordance with one of the foregoing claims, whereby the polymer structure of the branched polyglucane is based upon D-glucane monomers linked by 1,4-alpha-glycosidic units.

8. Procedure in accordance with one of the foregoing claims where the branched polyglucane has been chemically modified.

9. Procedure in accordance with one of the foregoing claims whereby an alpha-amylase-resistant polyglucane is used as the branched glucane.

10. Procedure in accordance with one of the foregoing claims whereby more than one branched polyglucane is used.

11. Procedure in accordance with one of the foregoing claims, whereby the solution and the precipitating agent are mixed at a temperature of between 20 and 50 °C and the resulting mixture is cooled to a temperature between plus 10 °C and minus 10 °C.

12. Procedure in accordance with claim 11, whereby the resulting mixture is cooled to a temperature in the range between plus 5°C and minus 5 °C.

13. Procedure in accordance with one of the foregoing claims whereby water or an aqueous medium is used as the precipitating agent.

14. Procedure in accordance with one of the foregoing claims whereby dimethylsulphoxide is used as the solvent.

15. Procedure in accordance with one of the foregoing claims, whereby the water-insoluble linear polysaccharide is a linear polyglucane.

16. Procedure in accordance with one of the foregoing claims, whereby poly(1,4-alpha-Dglucane) is used as the water-insoluble linear polysaccharide.

17. Procedure in accordance with one of the foregoing claims, whereby poly(1,3-beta-D-glucane) is used as the water-insoluble linear polysaccharide.

18. Procedure in accordance with one of the foregoing claims, whereby the water-insoluble inear polysaccharide is a chemically modified polysaccharide.

19. Procedure in accordance with claim 18, whereby the water-insoluble linear polysaccharide has been esterified and / or etherified in at least one of the positions which is not involved in the formation of the polymer chain and, preferably in position 2-, 3-and/or 6.

20. Spherical micro-particles with a smooth surface, i.e. whereby the surface of the microparticle contains depressions or clefts which do not exceed a maximum of 20% of the mean diameter of the micro-particles and which can be obtained by a procedure in accordance with one of claims 1 to 19.

21. Spherical micro-particles consisting wholly or partially of at least one water-insoluble branched polyglucane, which corresponds to the solubility classes 5 - 7 of the "Deutsches Arzneibuch (DAB)".

22. Spherical micro-particles in accordance with claim 21, whereby the degree of branching in the branched polyglucane is between 0.5% and a maximum of 8%.

23. Spherical micro-particles in accordance with one of claims 21 to 22 which in addition contain a water-insoluble linear polysaccharide.

24. Spherical micro-particles in accordance with one of claims 21 to 23, whereby the branched polyglucane is poly(1,4-alpha-D-glucane).

25. Spherical micro-particles in accordance with one of claims 21 to 24, whereby the water-insoluble linear polysaccharide is poly(1,4-alpha-D-glucane).

26. Use of spherical micro-particles in accordance with one of claims 20 to 25 to effect separation of mixtures of materials.

27. Use of spherical micro-particles in accordance with one of claims 20 to 25 as a filler in polymers.

28. Use of spherical micro-particles in accordance with one of claims 20 to 25 in diagnostic testing.

## Revendications

1. Procédé pour la préparation de microparticules sphériques, qui sont constituées entièrement ou en partie par au moins un polysaccharide insoluble dans l'eau, l'au moins un polysaccharide insoluble dans l'eau, qui correspond aux classes de solubilité 5-7 de la Pharmacopée allemande (DAB), étant dissous dans un solvant ou mélange de solvants, on introduit la solution formée dans un agent de précipitation ou mélange d'agents de précipitation, éventuellement on refroidit le mélange formé et on sépare les microparticules formées, **caractérisé en ce que** le polysaccharide insoluble dans l'eau est pris parmi un polyglucane contenant des ramifications avec un degré de ramification de 0,5 % à au maximum 8 % et un mélange d'un polyglucane contenant des ramifications et un polysaccharide linéaire, le taux de polyglucanes contenant des ramifications dans le mélange est d'au maximum 30 % en masse par rapport à la masse totale du polysaccharide et du polyglucane.

2. Procédé selon la revendication 1, le polysaccharide insoluble dans l'eau étant un mélange d'un polyglucane contenant des ramifications ayant un degré de ramification supérieur à zéro et d'au maximum 8 % et d'un polysaccharide linéaire.

3. Procédé selon la revendication 1 ou 2, où le polyglucane contenant des ramifications provient d'une source végétale.

4. Procédé selon la revendication 3, où la source végétale est l'amidon.

5. Procédé selon l'une des revendications 1 ou 2, où le polyglucane contenant des ramifications provient d'une source animale.

6. Procédé selon la revendication 5, où la source animale est le glycogène.

7. Procédé selon l'une des revendications précédentes, où la colonne polymère du polyglucane contenant des ramifications est constituée par des monomères de D-glucane reliés par des liaisons 1,4-alpha-glycosidiques.

8. Procédé selon l'une des revendications précédentes, où le polyglucane contenant des ramifications est modifié chimiquement.

9. Procédé selon l'une des revendications précédentes, où l'on emploie en tant que polyglucane contenant des ramifications un polyglucane résistant à l'alpha-amylase.

10. Procédé selon l'une des revendications précédentes, où l'on emploie un polyglucane contenant plus d'une ramification.

11. Procédé selon l'une des revendications précédentes, où l'on mélange la solution et l'agent de précipitation à une température dans le domaine de 20 à 50°C et on refroidit le mélange formé à une température dans le domaine de plus de 10°C à moins 10°C.

12. Procédé selon la revendication 11, le mélange formé étant refroidi à une température dans le domaine de plus 5°C à moins 5°C.

13. Procédé selon l'une des revendications précédentes, où l'on emploie l'eau ou un milieu aqueux en tant qu'agent de précipitation.

14. Procédé selon l'une des revendications précédentes, où l'on emploie le diméthylsulfoxyde en tant que solvant.

15. Procédé selon l'une des revendications précédentes, où le polysaccharide linéaire insoluble dans l'eau est un polyglucane linéaire.

16. Procédé selon l'une des revendications précédentes, où l'on emploie le poly(1,4-alpha-D-glucane) en tant que polysaccharide linéaire insoluble dans l'eau.

17. Procédé selon l'une des revendications précédentes, où l'on emploie le poly(1,3-bêta-D-glucane) en tant que polysaccharide linéaire insoluble dans l'eau.

18. Procédé selon l'une des revendications précédentes, où l'on emploie un polysaccharide chimiquement modifié en tant que polysaccharide linéaire insoluble dans l'eau.

19. Procédé selon la revendication 18, où le polysaccharide linéaire insoluble dans l'eau est estérifié et/ou ethérifié dans au moins une des positions qui ne participent pas à la formation de la chaîne polymère, de préférence en position 2, 3 et/ou 6.

20. Microparticules sphériques avec une surface lisse, c'est-à-dire que la surface des microparticules présente des creux ou bosses qui représentent au maximum 20 % du diamètre moyen des particules que l'on peut obtenir à l'aide d'un procédé selon l'une des revendications 1 à 19.

21. Microparticules sphériques constituées entièrement ou en partie par au moins un polyglucane insoluble dans l'eau contenant des ramifications, qui correspond aux classes de solubilité 5-7 de la Pharmacopée allemande (DAB).

22. Microparticules sphériques selon la revendication 21, où le degré de ramification du polyglucane contenant des ramifications étant de 0,5 % au maximum 8 %.

23. Microparticules sphériques selon l'une des revendications 21 à 22, renfermant en plus au moins un polysaccharide linéaire insoluble dans l'eau.

24. Microparticules sphériques selon l'une des revendications 21 à 23, où le polysaccharide linéaire insoluble dans l'eau est le poly(1,4-alpha-D-glucane).

25. Microparticules sphériques selon l'une des revendications 21 à 24, le polysaccharide linéaire insoluble dans l'eau est le poly(1,4-alpha-D-glucane).

26. Utilisation de microparticules sphériques selon l'une des revendications 20 à 25 pour la séparation de mélanges de matières.

27. Utilisation de microparticules sphériques selon l'une des revendications 20 à 25 comme charge dans des polymères.

28. Utilisation de microparticules sphériques selon l'une des revendications 20 à 25 dans des tests diagnostiques.
